**Europäisches Patentamt**

**European Patent Office**

**Office européen des brevets**

(19)

(11) Veröffentlichungsnummer: **0 044 948**
**A1**

(12) ## EUROPÄISCHE PATENTANMELDUNG

(21) Anmeldenummer: **81104908.9**

(22) Anmeldetag: **25.06.81**

(51) Int. Cl.³: **C 12 P 5/02, C 02 F 11/04**

(30) Priorität: **25.07.80 DE 3028212**

(43) Veröffentlichungstag der Anmeldung: **03.02.82**
**Patentblatt 82/5**

(84) Benannte Vertragsstaaten: **AT BE CH DE FR GB IT LI NL SE**

(71) Anmelder: **Howaldtswerke-Deutsche Werft Aktiengesellschaft Hamburg und Kiel, Schwentinestrasse, D-2300 Kiel 14 (DE)**

(72) Erfinder: **Harrendorf, Heinz, Ing. grad., Rhönweg 7, D-3000 Hannover 51 (DE)**

(74) Vertreter: **Kaiser, Henning, Howaldtswerke-Deutsche Werft Aktiengesellschaft Hamburg und Kiel Rossweg 20 Postfach 111 480, D-2000 Hamburg 11 (DE)**

(54) **Verfahren und Vorrichtung zur Erzeugung von Gas aus insbesondere landwirtschaftlichen Abfallstoffen.**

(57) Bei einem biologischen Verfahren und einer hierfür geeigneten Vorrichtung zur Erzeugung von Methangas aus landwirtschaftlichen Abfallstoffen mittels anaerober Bakterien in einem zylindrischen Reaktionsbehälter (1) wird bei der Zuleitung von Abfallstoffen über die Druckleitung (6) Gas aus dem Kopfraum (2) des Reaktionsbehälters (1) über die Leitung (8) hinzugefügt. Die Einleitung von Abfallstoff und Gas erfolgt über eine Injektordüse (7). Bei der Zuführung von frischem Abfallstoff aus der Sammelgrube (10) wird aus dem Reaktionsbehälter (1) über die Leitung (15) entnommene Flüssigkeit zugesetzt. Eine Zirkulation von flüssigem Abfallstoff aus dem Reaktionsbehälter (1) über Leitung (15), Pumpe (13), Wärmeaustauscher (16), Druckleitung (6) und Düse (7) ist möglich, um optimale Reaktionsbedingungen einzustellen und den Inhalt des Reaktionsbehälters (1) gut zu durchmischen.

Verfahren und Vorrichtung zur Erzeugung von Gas aus insbesondere landwirtschaftlichen Abfallstoffen

Die Erfindung bezieht sich auf ein Verfahren zur Erzeugung von Gas aus insbesondere landwirtschaftlichen Abfallstoffen. Die Erfindung betrifft auch die zur Durchführung des Verfahrens vorgesehene Vorrichtung.

Es ist bekannt, daß sich aus organischen Abfallstoffen auf biologische Weise mittels anaerober Bakterien Gas, z.B. Methangas, gewinnen läßt, das einem Aggregat, z. B. zur Heizung oder Stromerzeugung, zugeführt werden kann. Als Abfallstoff kommt in der Landwirtschaft insbesondere Gülle als Flüssigmist der Tierhaltung vor. Daneben fallen auch Pflanzenabfälle wie z. B. Rübenblätter an. Die Abfallstoffe werden in einem Reaktionsbehälter während einer Verweilzeit von 12 bis über 20 Tagen einem Umwandlungsprozess unterworfen. Die Prozesszeit kann z. B. bei Abwasserkläranlagen durch eine Umwälzung des Abfallstoffes in dem Reaktionsbehälter verkürzt werden, wozu Rühreinrichtungen vorgeschlagen wurden. Diese Rühreinrichtungen und auch rotierende Strahldüsen, die mit flüssigem Abfallstoff aus dem Behälter gespeist werden, sollen zugleich dazu dienen, die sich bei bestimmten Abfallstoffen oben auf der Flüssigkeit absetzende Schwimmschlammdecke zu zerstören.

Ein Nachteil dieser bekannten Rühreinrichtungen besteht darin, daß bei ihnen bewegte Teile in dem mit einem aggressiven Medium gefüllten Reaktionsbehälter angeordnet sind, so daß die Teile einem erhöhten Verschleiß unterliegen, aber nur schwierig auszuwechseln sind. Außerdem wird durch diese Vorrichtungen meist nur der obere Teil des Behälterinhaltes umgewälzt.

Der Erfindung liegt die Aufgabe zugrunde, den biologischen Prozess durch eine bessere Mischung der mehr oder weniger

0044948

umgewandelten Abfallstoffe zu verbessern, wobei gegebenenfalls auch eine Schwimmschlammdecke aufzubrechen ist, und die bessere Mischung im Reaktionsbehälter mittels einer fest anzuordnenden Einrichtung zu erreichen, die keine störungsanfälligen beweglichen Teile enthält.

Zur Durchführung des biologischen Verfahrens wird ein Reaktionsbehälter vorzugsweise in Form eines stehenden Zylinders verwendet, in den pumpfähiger Abfallstoff eingeleitet wird und in dessen Kopfraum sich das Gas sammelt. Der Abfallstoff wird vor seiner Einführung in den Behälter noch gegebenenfalls zerkleinert, temperiert und auch bezüglich z. B. des pH-Wertes konditioniert.

Zur Lösung der erfindungsgemäßen Aufgabe wird dem in den Behälter eingebrachten Abfallstoff ein Anteil von aus dem Kopfraum des Behälters abgezogenem Gas beigegeben. Der frische Abfallstoff strömt aus der Druckleitung etwa nach oben gerichtet in den Behälterinhalt, wobei seine Strömung durch austretende und aufsteigende Gasblasen unterstützt wird. Die Zuführung des Gases erfolgt in den Strom des in den Behälter gepumpten Abfallstoffes unmittelbar vor oder nach oder bei seinem Austreten aus der Druckleitung. Hierdurch wird in dem gesamten Behälterinhalt eine umwälzende Strömung erzeugt, durch die der zugeführte Abfallstoff und die in dem biologischen Prozess schon mehr oder weniger stark umgewandelten und entgasten Bestandteile gemischt werden. Die aufsteigenden Gasblasen nehmen kleine, frisch gebildete Bläschen mit und durchstoßen die Oberfläche des Behälterinhaltes. Dabei durchbrechen und zerstören sie auch eine etwa vorhandene Schwimmschlammdecke.

Für die Einspeisung des Gases in den unteren Teil des Reaktionsbehälters wird wenigstens eine Düse vorgesehen. Vorzugsweise wird eine Injektordüse verwendet, die nach dem Prinzip der Wasserstrahlpumpe bei der Zuführung des flüssigen Abfallstoffes in den Behälter Gas aus dem Kopfraum

0044948

- 3 -

des Behälters bzw. aus einer mit dem Kopfraum in Verbindung stehenden Leitung absaugt.

In weiterer Ausgestaltung der Erfindung wird dem zugeführten frischen Abfallstoff bereits vor der Einleitung in den Reaktionsbehälter ein Anteil von anaerobe Bakterien enthaltender Flüssigkeit aus dem Behälter zugemischt. Diese Mischung wird außerhalb des Behälters auf eine Temperatur, einen pH-Wert usw. konditioniert, um auf diese Weise in dem Behälter optimale Prozessbedingungen, z. B. eine Temperatur von 35 $^{o}$C, zu erzeugen.

Zu diesem Zweck ist unterhalb des im Betrieb niedrigsten Flüssigkeitsspiegels am Behälter eine Entnahmeleitung angeordnet, die zu einer Umwälzpumpe führt und in die die Zuleitung für aus der Sammelgrube kommenden frischen Abfallstoff mündet. Die Mischung von frischem und teilweise umgewandeltem Abfallstoff wird über einen Wärmeaustauscher usw. in einer Druckleitung zur Injektordüse in dem Behälter gepumpt. Hierdurch wird der frische Abfallstoff schon während seiner Zuführung in den Behälter mit anaeroben Bakterien geimpft.

Auch ohne Zuführung frischen Abfallstoffes kann der vorstehend geschilderte Kreislauf über Entnahmeleitung, Umwälzpumpe und Druckleitung benutzt werden, um den Behälterinhalt zu durchmischen und z. B. die Temperatur im Behälter mittels des Wärmetauschers und/oder den pH-Wert durch Zugabe von sauren oder alkalischen Mitteln zu korrigieren. In jedem Falle wird bei Austritt von Flüssigkeit aus einer Injektordüse auch Gas angesaugt und in den Behälterinhalt eingeblasen.

Weitere Einzelheiten werden bei einem Ausführungsbeispiel anhand der beigefügten schematischen Zeichnung beschrieben.

BAD ORIGINAL

Der Reaktionsbehälter 1 ist ein stehender Zylinder, dessen Außenseite mit einer Wärmedämmschicht versehen ist. In dem Behälter 1 befindet sich der mehr oder weniger flüssige Abfallstoff während des Prozesses. Im oberen Kopfraum 2 sammelt sich das entstehende Gas, das über eine Leitung 3 entnommen werden kann. Am unteren Ende 4 sammeln sich Schlamm, Sand und andere nicht mehr für den Prozess nutzbare Stoffe, die periodisch über eine Schlammabzugsleitung 5 oder ähnliche Einrichtungen entfernt werden.

Über eine Druckleitung 6 wird dem Reaktionsbehälter 1 Abfallstoff zugeführt, wobei dieser Abfallstoff über den Diffusor einer Injektordüse 7 nach oben gerichtet in den Behälterinhalt austritt. Der Flüssigkeitsstrom zieht dabei in der Düse 7 Gas über die Leitung 8 aus der Leitung 3 bzw. dem Kopfraum 2. Mit dem Flüssigkeitsstrom steigen Gasblasen nach oben und fördern die Durchmischung und eine Zirkulation des Behälterinhaltes. Die verhältnismäßig große aufsteigende Gasmenge zerstört auch eine etwa auf der Flüssigkeit schwimmende Schlammdecke, bevor das Gas in den Kopfraum 2 austritt. Aus dem Kopfraum 2 gelangt das Gas über die Leitungen 3 und 9 sowie über nicht dargestellte Filter und Trocknungseinrichtungen zu einem ebenfalls nicht gezeigten Speicher, aus dem es nach Bedarf entnommen werden kann.

Gülle und andere Abfallstoffe werden in einer Sammelgrube 10 zunächst gesammelt. Diese Grube kann mit einem Rührwerk 11 versehen sein, das eine annähernd gleichmäßige Mischung erzeugt. Eine Tauchpumpe 12, der gegebenenfalls noch ein Zerkleinerer vorgeschaltet ist, fördert in regelmäßigen Zeitabständen oder nach Bedarf die Abfallstoffe über eine Leitung 14 zu einer Umwälzpumpe 13. Zur Umwälzpumpe 13 führt auch eine Leitung 15, die vorzugsweise zwischen einem Viertel und der Hälfte der Höhe der Flüssigkeitssäule in dem Reaktionsbehälter 1 beginnt und durch die anaerobe Bakterien von hoher Aktivität enthaltender teilweise umgewandelter, flüssiger Abfallstoff aus dem Behälter abgezogen werden kann. Diese Flüssigkeit wird mit frischem Abfallstoff in der Pumpe 13 und dem anschließenden System gemischt. Die Mischung fließt durch einen Wärmeaustauscher 16 und die Druckleitung 6 zur Düse 7, wobei beispielsweise über die Leitung 17 Zusätze zur Beeinflussung des pH-Wertes mittels einer Dosiereinrichtung eingespeist werden können.

Der Reaktionsbehälter 1 besitzt wenigstens ein nicht gezeichnetes Mannloch, um die Düse 7 zu montieren. Da die Düse keine beweglichen Teile enthält und aus korrosionsfestem Material hergestellt sein kann, sind Wartungsarbeiten im Behälter praktisch nicht notwendig. Sollte die Düse verstopft werden, so kann sie von außen über den Anschluß 29 mit höherem Druck durchgespült oder durchgeblasen werden. In der Leitung 8 kann auch eine den Druck des zugeführten Gases erhöhende Pumpe angeordnet werden, die nicht dargestellt ist.

Die Form der Injektordüse 7 wird so gewählt, daß ein genügend starker Flüssigkeitsstrahl und eine ausreichende Gasmenge austreten, wobei aber die notwendige Leistung der Umwälzpumpe 13 nicht außergewöhnlich hoch sein soll. Die Injektordüse 7 ist somit der Größe des Behälters 1 anzupassen, die etwa zwischen 30 $m^3$ und 400 $m^3$ betragen kann und von der Menge der anfallenden Abfallstoffe abhängt. Bei Behältern mit großem Querschnitt und/oder großem Volumen bis z. B. 4000 $m^3$ können auch mehrere Düsen verwendet werden. Die Injektordüse 7 kann lotrecht oder in einer geneigten Stellung etwa nach oben gerichtet angeordnet werden.

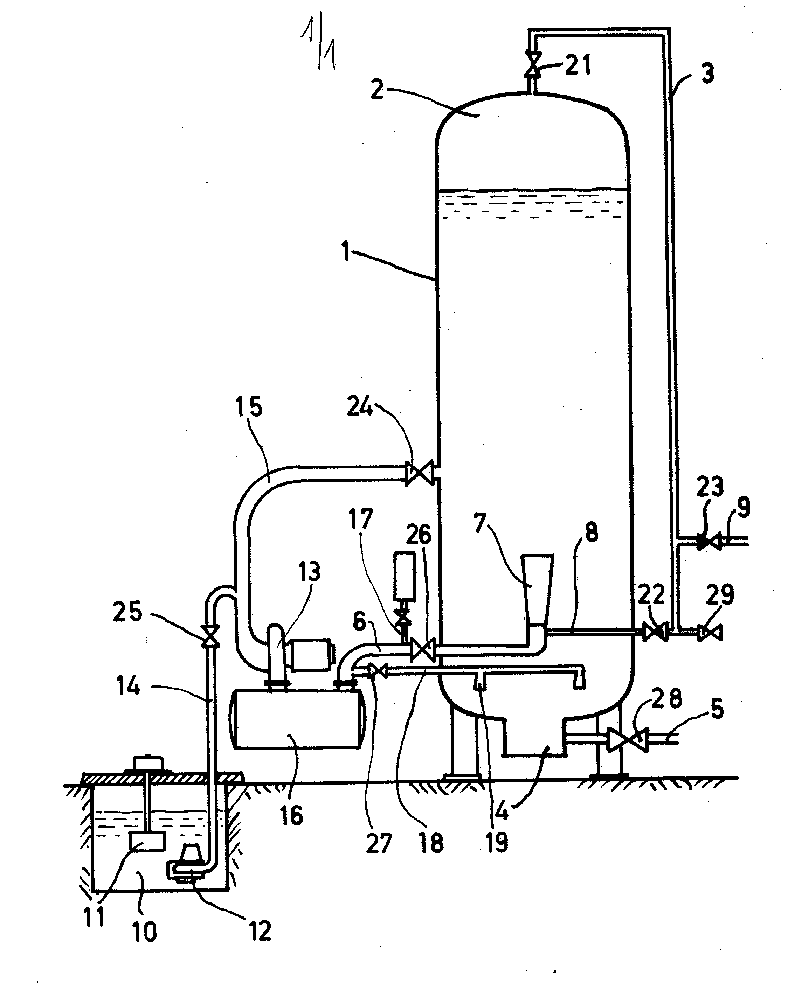

Zwischen den einzelnen Teilen der Anlage befinden sich, wie auf der Zeichnung angedeutet, Absperrorgane oder Ventile, durch die auch die Durchflußmengen geregelt werden können. Ventil 21 in der Gasleitung 3 kann den Kopfraum 2 abschliessen. Ventil 22 in Leitung 8 regelt die Gaszufuhr zur Düse 7. Ventil 23 schließt die weiterführende Gasleitung 9 ab. Mit Ventil 24 wird die Menge des durch die Entnahmeleitung 15 umgepumpten, teilweise zersetzten Abfallstoffes geregelt. Ventil 25 verschließt die Zuleitung 14, wenn Abfallstoff nur durch die Leitungen 15 und 6 umgepumpt wird. Ventil 26 regelt die Menge des durch die Druckleitung 6 zur Düse 7 gelangenden Abfallstoffes. Ventil 28 wird geöffnet, um den sich unten im Behälter absetzenden Schlamm über die Leitung 5 abzuziehen. Weitere nicht gezeigte Regel- oder Absperreinrichtungen können zusätzlich angewendet werden. Die Anlage ist ferner mit nicht gezeigten Sicherheitseinrichtungen und mit verschiedenen Meß- und Kontrolleinrichtungen versehen, um in dem Reaktionsbehälter optimale Bedingungen aufrechtzuerhalten und das Verfahren wenigstens teilweise automatisch ablaufen zu lassen.

Durch Umwälzen von Behälterflüssigkeit über Leitung 15, Pumpe 13 und Wärmeaustauscher 16 kann gegebenenfalls auch ohne Zufügung von frischem Abfallstoff die Temperatur im Behälter erhöht werden, wenn dies für eine andere Bakterienart erforderlich sein sollte. Bei jedem Umpumpen über Umwälzpumpe 13 und Injektordüse 7 wird zugleich auch die Reaktion in dem Behälter mittels Durchmischung und Einblasen von Gas angeregt und der Prozess aktiviert und beschleunigt, und die sich aus leichten, festen Bestandteilen auf der Flüssigkeit absetzende Schwimmschlammdecke zerbrochen, so daß auch deren Teile in dem biologischen Prozess in der Flüssigkeit abgebaut werden können.

Von der Druckleitung 6 kann eine Zweigleitung 18 mit einem Ventil 27 abzweigen, die zu Austrittsdüsen 19 unten in dem Reaktionsbehälter 1 führt. Die Düsen 19 sind gegen den Boden des Behälters 1 gerichtet. Mit ihnen ist gegebenenfalls eine gelegentliche Auflockerung des sich im unteren Teil des Behälters 1 absetzenden, weitgehend schon entgasten Abfallstoffes möglich. Dabei wird zweckmäßigerweise schon teilweise abgebauter Abfallstoff umgepumpt.

Durch das vorliegende Verfahren und die zu seiner Durchführung vorgesehene Vorrichtung wird die Erzeugung von Gas, insbesondere Methangas, aus einer biologischen Masse von organischen Bestandteilen im Hinblick auf eine optimale Gesamtausbeute verbessert. Die bessere Durchmischung, die nicht nur durch die Zufügung von frischem Abfallstoff und das Umpumpen von teilweise abgebauten Stoffen, sondern insbesondere auch durch das Einbringen von in dem Verfahren entwickeltem Gas erfolgt, schafft laufend neue Kontaktflächen in der organischen Masse für die Bakterien. Innerhalb einer bestimmten Zeit ist daher der Abbau der Masse vollständiger möglich.

Patentansprüche

1. Verfahren zur Erzeugung von Gas aus insbesondere landwirschaftlichen Abfallstoffen mittels anaerober Bakterien, wobei ein pumpfähiger Abfallstoff konditioniert in einen Reaktionsbehälter gepumpt wird, aus dessen Kopfraum das entstehende Gas abgeleitet wird, dadurch gekennzeichnet, daß dem Abfallstoff bei seiner Einleitung in den Reaktionsbehälter ein Teil des Gases aus dem Kopfraum zugeführt wird.

2. Verfahren nach Anspruch 1, dadurch gekennzeichnet, daß dem in den Reaktionsbehälter gepumpten frischen Abfallstoff ein Anteil von Flüssigkeit aus dem Behälter zugeführt wird und die in den Behälter einzuleitende Flüssigkeitsmischung auf eine Temperatur, einen pH-Wert usw. konditioniert wird, um in dem Behälter optimale Prozessbedinungen einzustellen.

3. Vorrichtung zur Erzeugung von Gas aus insbesondere landwirtschaftlichen Abfallstoffen gemäß einem Verfahren nach Anspruch 1 oder 2, bestehend aus einem wärmeisolierten, stehenden Reaktionsbehälter, einer Sammelgrube für Abfallstoffe, wenigstens einer Pumpe, einem Wärmeaustauscher und Einrichtungen zum Ableiten des Gases und zum Abziehen des Schlammes aus dem Reaktionsbehälter, dadurch gekennzeichnet, daß im unteren Teil des Reaktionsbehälters (1) wenigstens eine Düse (7) zur Einleitung von in dem biologischen Verfahren erzeugten Gas in die flüssigen Abfallstoffe angeordnet ist.

4. Vorrichtung nach Anspruch 3, dadurch gekennzeichnet, daß die Düse (7) eine Injektordüse ist, durch die die flüssigen Abfallstoffe in den Reaktionsbehälter (1) gedrückt und zugleich Gas aus einer mit dem Kopfraum (2) des Behälters (1) in Verbindung stehenden Leitung (3, 8) angesaugt und dem Abfallstoff zugemischt werden kann.

5. Vorrichtung nach Anspruch 3 oder 4, dadurch gekennzeichnet, daß die Düse (7) im unteren Teil des Reaktionsbehälters (1) angeordnet und nach oben gerichtet ist.

6. Vorrichtung nach einem der Ansprüche 3 bis 5, dadurch gekennzeichnet, daß unterhalb des Flüssigkeitsspiegels im Reaktionsbehälter (1) eine Entnahmeleitung (15) beginnt und über eine Umwälzpumpe (13) und einen Wärmeaustauscher (16) als Druckleitung (6) zur Düse (7) im Reaktionsbehälter (1) führt und mit der Zuleitung (14) für frischen Abfallstoff verbindbar ist.

7. Vorrichtung nach Anspruch 6, dadurch gekennzeichet, daß die Entnahmeleitung (15) unterhalb etwa der halben Höhe der in dem Reaktionsbehälter (1) befindlichen Flüssigkeit beginnt.

8. Vorrichtung nach Anspruch 6 oder 7, dadurch gekennzeichnet, daß von der Druckleitung (6) eine Zweigleitung (18) zu gegen den Boden des Reaktionsbehälters (1) gerichteten Austrittsdüsen (19) führt.

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>DE - A - 1 584 902</u> (FMC CORP.)<br><br> + Ansprüche 1,6; Seiten 18,19; Seite 20, Abs. 1; Fig. 2 +<br><br>-- | 1,3,4, 5,6 |
| | W. BAADER, E. DOHNE, M. BRENN-DÖRFER "Biogas in Theorie und Praxis", KTBL-Schrift 229, 1978<br><br>KTBL-SCHRIFTEN-VERTRIEB IM LAND-WIRTSCHAFTSVERLAG GMBH, Münster-Hiltrup<br><br> + Seite 34, Fig. 11; Seite 40, Zeilen 19-37 +<br><br>---- | 1,3,5 |

**KLASSIFIKATION DER ANMELDUNG (Int. Cl.³)**

C 12 P 5/02
C 02 F 11/04

**RECHERCHIERTE SACHGEBIETE (Int. Cl.³)**

C 12 P
C 02 F

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

**X** Der vorliegende Recherchenbericht wurde für alle Patentansprüche erstellt.

| Recherchenort | Abschlußdatum der Recherche | Prüfer |
|---|---|---|
| WIEN | 10-09-1981 | WOLF |

EPA form 1503.1 06.78